# EUROPÄISCHE PATENTSCHRIFT

(11) **EP 1 807 214 B1**
(45) Veröffentlichungstag und Bekanntmachung des Hinweises auf die Patenterteilung: **30.01.2013**
(21) Anmeldenummer: 05798766.1
(22) Anmeldetag: 27.10.2005
(51) Int. Cl.: B03C 3/155, B03C 3/011, B03C 3/019

(54) **VERFAHREN ZUM HERAUSFILTERN VON GERÜCHEN AUS EINER LUFTSTRÖMUNG UND FILTERVORRICHTUNG MIT EINEM GERUCHSFILTER**
METHOD FOR FILTERING ODORS OUT OF AN AIR FLOW, AND FILTERING DEVICE EQUIPPED WITH AN ODOR FILTER
PROCEDE POUR EXTRAIRE PAR FILTRATION DES ODEURS D'UN FLUX D'AIR ET DISPOSITIF DE FILTRATION MUNI D'UN FILTRE ANTI-ODEURS

(30) Priorität: 30.10.2004 DE 102004053030
(43) Veröffentlichungstag der Anmeldung: 18.07.2007
(73) Patentinhaber: Langner, Manfred H., 49509 Recke (DE)
(72) Erfinder: Langner, Manfred H., 49509 Recke (DE)
(74) Vertreter: Katscher Habermann Patentanwälte
(86) Internationale Anmeldenummer: PCT/EP2005/011526
(87) Internationale Veröffentlichungsnummer: WO 2006/048183

(56) Entgegenhaltungen:
- EP-A- 0 049 454
- EP-A1- 0 785 016
- DE-A1- 2 028 153
- DE-A1- 3 618 403
- DE-A1- 3 901 663
- DE-A1- 10 113 548
- US-A- 5 622 543

## Beschreibung

Die Erfindung betrifft ein Verfahren und eine Vorrichtung zum Herausfiltern von Gerüchen aus einem Luftstrom, wobei ein Geruchsstoffe enthaltender Luftstrom durch eine Filtervorrichtung mit einem Geruchsfilter hindurchgeführt wird und der Geruchsfilter Aktivkohle oder dergleichen als Filtermaterial aufweist, wobei in der Luftströmung vor dem Eintritt in den Geruchsfilter ein Plasma erzeugt wird und dass zumindest ein Teil der in der Luftströmung enthaltenen Teilchen vor dem Eintritt in den Geruchsfilter dissoziiert wird.

Filtervorrichtungen mit einem Geruchsfilter werden beispielsweise in Abluftsystemen regelmäßig dann eingesetzt, wenn die abzuführende Abluft große Mengen an Geruchsstoffen enthält und diese zumindest in der vorliegenden Konzentration nicht unmittelbar der Umwelt zugeführt werden sollen. Typische Anwendungsgebiete für Geruchsfilter bestehen regelmäßig im Zusammenhang mit der Erwärmung und Zubereitung von Lebensmitteln. Auch in der chemischen oder farbenverarbeitenden Industrie werden oftmals Geruchsfilter eingesetzt.

Je nach den geforderten Filtereigenschaften können verschiedene Filterverfahren wie beispielsweise Lösungsmittelspülen, elektrostatische Filterverfahren, Plasmafilter oder, wie in den meisten Fällen, Aktivkohle enthaltende Filtermaterialien angewendet werden.

In EP 0 785 016 A1 wird ein Verfahren zur industriellen Filterung von schädlichen Gasen beschrieben, bei dem ein bereits bekannter biologischer Reinigungsschritt mit einer nicht-thermischen Plasmareinigung kombiniert. Die biologischen Reinigungsverfahren wie beispielsweise Biowäscher oder Festbett-Biofilteranlagen eignen sich nur für gewerbliche und dauerhaft betriebene Reinigungsvorrichtungen, wobei in geringer Konzentration enthaltene gasförmige Schadstoffe mit Hilfe von Mikroorganismen umgewandelt und abgebaut werden. Durch die Kombination mit einer nicht-thermischen Plasmareinigung können auch diejenigen gasförmigen Verbindungen aus der Abluft herausgefiltert und deren Anteil reduziert werden, die mit den bekannten biologischen Reinigungsschritten nicht oder nur in eingeschränktem Maße herausgefiltert werden können.

Beispielsweise in den Küchen von Privathaushalten können Umluft-Dunztabzugshauben eingesetzt werden, bei welchen die über der Kochstelle entstehende Abluft angesaugt, durch ein Filtersystem geführt und danach wieder dem Raum, in welchem sich die Kochstelle befindet, zugeführt wird. Neben oftmals mechanisch ausgeführten Filtervorrichtungen, mit welchen kleine Partikel wie Fetttröpfchen oder Wasserdampftropfen aus der Abluft abgeschieden werden können, können Umluft-Dunstabzugshauben vorteilhafterweise zusätzliche Geruchsfilter aus Aktivkohle oder einem ähnlichen Filtermaterial aufweisen. Mit dem Geruchsfilter soll vermieden werden, dass in der Abluft enthaltene Geruchsstoffe, die oftmals von den Filtersystemen für Fetttröpfchen und Wasserdampf nicht erfasst werden können, die Dunstabzugshaube ungehindert passieren können und wieder unmittelbar an die Umgebung abgegeben werden.

Die meisten bekannten Geruchsfilter basieren auf einem nicht oder nur sehr aufwendig regenerierbaren Filtermaterial, wie beispielsweise Aktivkohle oder dergleichen, dessen Filterwirkung mit der Zeit üblicherweise nachlässt, weshalb der Geruchsfilter in Abständen gewechselt bzw. ersetzt werden muss. Während die zeitlichen Abstände für den erforderlichen Wechsel eines Geruchsfilters und die damit verbundenen Kosten eine auch wirtschaftlich sinnvolle Nutzung bei Dunstabzugshauben ermöglichen, die für einen Einsatz in Privathaushalten vorgesehen sind, erfordern gewerblich oder industriell genutzte Dunstabzugshauben oftmals große Mengen an Filtermaterial, welches in kurzen Zeitabständen ausgetauscht und erneuert werden muss.

Der Wirkungsgrad der Geruchsfilter kann üblicherweise nur durch eine Zunahme des Filtermaterials, bzw. eine Vergrößerung des Geruchsfilters erhöht werden. Oftmals lässt sich bei gewerblich genutzten Dunstabzugshauben beispielsweise in Restaurants oder in Großküchen auf Grund des hohen Anteils an Geruchsstoffen in der Abluft keine zufriedenstellende Filterwirkung für Geruchsstoffe erreichen, ohne dass der auf Grund der Menge des Filtermaterials bewirkte Strömungswiderstand zu einer unerwünschten, nicht mehr hinnehmbaren Minderung des Abluft-Fördervolumens führt.

Aufgabe der Erfindung ist es demzufolge, ein Verfahren zum Herausfiltern von Geruchsstoffen aus einem Luftstrom so auszugestalten, dass der Wirkungsgrad verbessert wird. Dabei sollte möglichst durch das Filterverfahren der Strömungswiderstand bei dem Hindurchführen der Luft nicht erheblich verschlechtert werden.

Diese Aufgabe wird erfindungsgemäß dadurch gelöst, dass der Luftstrom zuerst durch einen Partikelfilter hindurchgeführt wird, bevor eine zumindest teilweise Ionisation und/oder Dissoziation der darin enthaltenen Teilchen bewirkt wird anschließend der Luftstrom in den Geruchsfilter eintritt. Durch die Vorschaltung eines Partikelfilters wird verhindert, dass grobe Verunreinigungen in die nachfolgenden Filterabschnitte eindringen und zu einer vorschnellen Sättigung des nicht oder nur mit großem Aufwand regenerierbaren Filtermaterials bzw. zu einer Beeinträchtigung der Filterwirkung führen können. Auch kann dadurch vermieden werden, dass ein unerwünscht hoher Anteil der für eine Ionisierung bzw. Dissoziation der Geruchsstoffe zugeführten Energie durch Partikel, insbesondere Staubteilchen oder Fetttröpfchen absorbiert wird und nicht zum Herausfiltern von Geruchsstoffen zur Verfügung steht.

Das erfindungsgemäße Verfahren kann nicht nur zur Filterung von Gerüchen aus einem Abluftstrom eingesetzt werden, sondern auch zur Filterung eines Luftstroms im Umluftbetrieb oder zur Filterung von Zuluft für Räumlichkeiten mit besonderen Anforderungen an den Reinheitsgrad der Raumluft.

Es hat sich herausgestellt, dass die erfindungsgemäße Kombination dieser Filterverfahren eine deutlich verbesserte Filterwirkung aufweist, als es durch die getrennte Verwendung der einzelnen Filterverfahren nahegelegt würde.

Entweder infolge einer unmittelbaren Dissoziation oder aber verursacht durch die Ionisation und anschließende Reaktion werden Geruchsstoffe, bzw. Geruchsmolektile in gegebenenfalls geruchsneutrale Reaktionsprodukte umgewandelt. Solche geruchsneutralen Reaktionsprodukte müssen nicht mehr unbedingt aus dem Luftstrom herausgefiltert werden, um unerwünschte Gerüche zu vermeiden. Zudem begünstigt das Ansammeln und Zurückhalten von energetisch angeregten Reaktionsprodukten gemeinsam mit noch im Luftstrom befindlichen Geruchsstoffen auf Grund der längeren Verweildauer in dem nachfolgenden Geruchsfilter eine weitere Reaktion mit Geruchsstoffen, die zumindest teilweise ebenfalls in geruchsneutrale Reaktionsprodukte umgewandelt werden. Lediglich die verbleibende, erheblich reduzierte Menge der ursprünglich vorhandenen Geruchsstoffe muss durch den Geruchsfilter aus dem Luftstrom abgeschieden und herausgefiltert werden. Eine unerwünschte Sättigung des aktivkohlehaltigen Geruchsfilters wird dadurch zeitlich verzögert, so dass bei geringerem Wartungsaufwand eine zusätzlich verbesserte Filterwirkung eintritt.

Es ist vorgesehen, dass in dem Luftstrom vor dem Eintritt in den Geruchsfilter ein Plasma erzeugt wird oder elektrische Entladungen erzeugt werden.

In praktischen Versuchen wurde die Geruchsstoffkonzentration in der Abluft direkt über mehreren Friteuseeinheiten eines Schnellrestaurants mit der Geruchsstoffkonzentration der Abluft verglichen, nachdem die Abluft einem erfindungsgemäßen Filterverfahren unterworfen wurde. Während sowohl die Filterwirkung eines Aktivkohle-Geruchsfilters als auch die Filterwirkung eines Plasma-basierenden Geruchfilters jeweils eine Minderung der Geruchsstoffkonzentration um den mittleren Faktor 3 bis 5 erreichen konnte, ließ sich mit der erfindungsgemäßen Kombination einer Filterung mittels eines Geruchsfilters mit Aktivkohle in Verbindung mit einer vorausgehenden Ionisation bzw. Dissoziation zumindest von einen Teil der im Abluftstrom enthaltenen Teilchen und insbesondere der Geruchsstoffe eine Geruchsminderung um den Faktor 50 bis weit über 100 erreichen.

Die ionisierten oder dissoziierten Geruchsstoffe lassen sich wesentlich wirkungsvoller mit einem Filtermaterial wie beispielsweise Aktivkohle aus dem Luftstrom herausfiltern, als es ohne die zusätzliche Ionisierung bzw. Dissoziation möglich wäre. Gleichzeitig wird die Aktivkohle nur in einem geringeren Maße verbraucht bzw. ein Aktivkohlefilter mit der Zeit in seiner Wirkung vergleichsweise langsamer herabgesetzt, so dass trotz einer verbesserten Filterwirkung für Geruchsstoffe die zeitlichen Abstände eher länger werden, in denen ein Auswechseln und Erneuern des Filtermaterials aus Aktivkohle erforderlich ist.

Es ist auch seit längerem bekannt, dass insbesondere kleine Partikel, die mit üblichen mechanischen Filtervorrichtungen nicht oder nur ungenügend aus der Abluft entfernt werden können, durch die Erzeugung eines Plasmas in der Abluft in einfacher Weise separiert und abgeschieden werden können. Es sind verschiedene Verfahren zur Filterung durch Plasma-Erzeugung bekannt, welche beispielsweise auf der Erzeugung von atmosphärischem Plasma oder auf einer dielektrisch behinderten Entladung zwischen zwei flächigen Elektroden beruhen.

In dem Aktivkohle-Filtermaterial werden dann auch angeregte, ionisierte oder dissoziierte Teilchen bzw. Moleküle aufgefangen, so dass eine weitere Reaktion bislang nicht oxidierter Geruchsmoleküle bzw. Geruchsstoffe stattfindet, wodurch die Filterwirkung zusätzlich verbessert wird.

Zusätzlich entsteht bei den genannten Verfahren zur Ionisierung bzw. Dissoziation der in dem Luftstrom enthaltenen Teilchen oftmals Ozon. Das Ozon reagiert innerhalb kurzer Zeit mit anderen in dem Luftstrom enthaltenen Teilchen, wobei eine Oxidationsreaktion stattfinden kann. Das Ozon wird zusammen mit anderen Teilchen in dem Aktivkohle-Fitermaterial zurückgehalten und kann dort mit weiteren Geruchsstoffen reagieren und diese in geruchsneutrale Stoffe zersetzen. Eine erhebliche Verbesserung der Filterwirkung wird demzufolge auch durch die Erzeugung, bzw. ganz allgemein durch die Bereitstellung von Ozon im Abluftstrom unmittelbar vor dem Aktivkohle-Filtermaterial bewirkt.

Zudem wird bei der Reaktion von Ozon mit anderen im Aktivkohle-Filtermaterial zurückgehaltenen Molekülen Reaktionsenergie freigesetzt, die in Abhängigkeit von der stattfindenden Reaktion eine Regeneration des Aktivkohle-Materials bewirken oder zumindest begünstigen kann. Das Aktivkohle-Filtermaterial wird dadurch während des Filtervorgangs gleichzeitig teilweise gereinigt, bzw. eine zunehmende Verringerung der Filterwirkung zumindest zeitlich verzögert.

Die Erfindung betrifft auch eine Filtervorrichtung für einen Luftstrom mit einem Geruchsfilter, wobei der Geruchsfilter Aktivkohle oder dergleichen als Filtermaterial aufweist und wobei die Filtervorrichtung eine Energie zuführende Vorrichtung zur Erzeugung eines Plasmas zur Dissoziation der in dem Luftstrom enthaltenen Teilchen aufweist, die entlang der Luftströmung vor dem Geruchsfilter angeordnet ist.

Erfindungsgemäß ist vorgesehen, dass die Filtervorrichtung einen mechanischen Partikelfilter aufweist, der in Strömungsrichtung vor der Energie zuführenden Vorrichtung und dem nachfolgenden Geruchsfilter angeordnet ist. Mit einem solchen groben Partikelfilter, beispielsweise einem Fettabscheider oder Prallabscheider, kann in einfacher Weise bereits ein Großteil der in der Luftströmung mitgeführten Verunreinigungen herausgefiltert und abgeschieden werden.

In Abhängigkeit von der geplanten Verwendung und den im Voraus bekannten Anforderungen der Filtervorrichtung kann der Partikelfilter gegebenenfalls mehrstufig ausgeführt sein, um stufenweise eine Abscheidung von feinen und feinsten Teilchen und Verunreinigungen im Luftstrom zu erreichen. Der Partikelfilter kann auch eine zusätzliche Beregnungs- oder eine Wäschereinheit mit einem Sprühnebel von Wasser oder Reinigungsmaterial aufweisen.

Besonders vorteilhafterweise ist vorgesehen, dass die Energie zuführende Vorrichtung zwei im Abstand zueinander angeordnete Elektroden aufweist, zwischen denen eine Wechselspannung anlegbar ist. Durch die Abmessung und Formgebung der Elektroden sowie die Ansteuerung der zwischen den Elektroden angelegten Wechselspannung lässt sich die Ionisation oder Dissoziation der mit der Luft zwischen den Elektroden durchströmenden Teilchen über einen weiten Bereich hinweg vorgeben und dadurch an die jeweiligen Anforderungen bzw. an die in der Luftströmung in besonders hoher Konzentration enthaltenen Geruchsstoffe anpassen.

Gemäß einer vorteilhaften Ausgestaltung des Erfindungsgedankens ist vorgesehen, dass die Energie zuführende Vorrichtung zwei im Abstand zueinander angeordnete Elektroden aufweist, zwischen denen eine pulsierende Gleichspannung oder eine Gleichspannung mit einem überlagerten Wechselspannungsanteil anlegbar ist. Die Elektroden können vorzugsweise als im Wesentlichen ebene Platten oder als ebene Gitter ausgestaltet sein. Es ist auch denkbar, mehrere im Abstand übereinander angeordnete Elektroden in dem Luftstrom anzuordnen.

Es ist vorgesehen, dass zwischen den Elektroden ein die Entladung behinderndes dielektrisches Material angeordnet ist. Es ist seit längerem bekannt, dass eine dielektrisch behinderte Entladung, wie sie durch die Anordnung eines Dielektrikums bzw. eines Isolators zwischen den Elektroden herbeigeführt wird, zu einer merklich verbesserten Ionisierung und Dissoziation und auch zu einer einfacheren und wirkungsvolleren Erzeugung und Aufrechterhaltung eines Plasmas führt.

Im einfachsten Fall ist jeweils eine von zwei Elektroden mit einer Isolationsschicht versehen.

Besonders vorteilhafterweise ist vorgesehen, dass der Partikelfilter ein mindestens teilweise offenporiger, hydrophiler Schaumstoff ist. Es hat sich gezeigt, dass derartige Filtermaterialien eine sehr gute Filterwirkung für Fett- und Wasserdampftröpfchen aufweisen und gleichzeitig die herausgefilterten Verunreinigungen sehr effizient aus dem Filtermaterial ausgewaschen und abgeführt werden können. Sofern die Luftströmung nicht ausschließlich trockene, staubige Verunreinigungen, sondern auch einen ausreichenden Anteil an Wasserdampf und Flüssigkeitströpfchen mit sich führt, reicht die in dem offenporigen, hydrophilen Schaumstoff absorbierte Flüssigkeitsmenge aus, um den weitgehend größten Anteil aller im Filtermaterial aufgefangenen Verunreinigungen von selbst aus dem Filtermaterial auszuwaschen, so dass die unter dem offenporigen, hydrophilen Schaumstoff aufgefangenen und gesammelten Verunreinigungen in einfacher Weise und mit geringem Aufwand abgeführt werden können.

Es hat sich gezeigt, dass das Herausfiltern größerer Teilchen, wie beispielsweise Dampf und Fetttröpfchen mit einem Durchmesser von 1 Mikrometer oder mehr dazu führt, dass die nachfolgend angeordnete Energie zuführende Vorrichtung und der Geruchsfilter auch über einen langen Zeitraum kaum verunreinigt und ihrer Wirkungsweise beeinträchtigt werden. Eine so ausgestaltete Filtervorrichtung lässt sich demzufolge auch in gewerblich und industriell genutzten Dunstabzugshauben verwenden, ohne dass ein unangemessen hoher Wartungsbedarf notwendig wäre. Mit einer solchen Kombination von einzelnen Filterelementen lassen sich nicht nur größere Teilchen wie Fetttröpfchen oder Wasserdampf, sondern auch sehr kleine, mechanisch kaum noch herausfilterbare Teilchen und Geruchsstoffe nahezu vollständig aus der Luftströmung herausfiltern.

Für den Einsatz bei Luftströmungen, welche eine erhebliche Staubfracht aufweisen, kann es zweckmäßig sein, als Partikelfilter einen Staubfilter vorzuschalten. Ein Staubfilter kann beispielsweise aus einem geeigneten Vlies oder Gelege aus textilen oder metallischen Materialien bestehen.

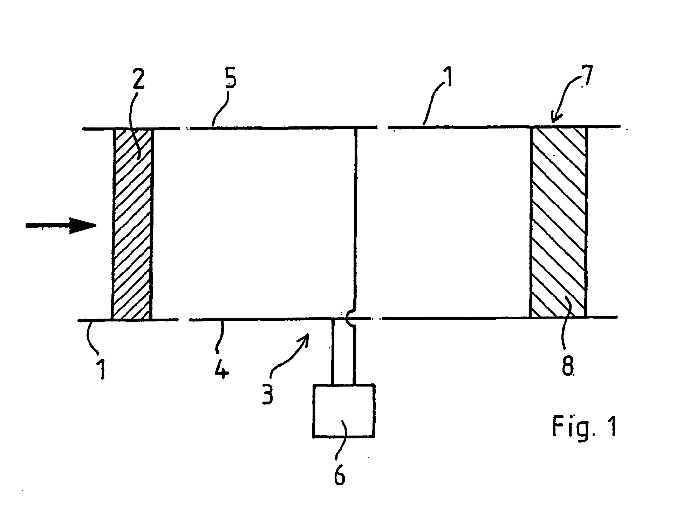

Nachfolgend wird schematisch ein Ausführungsbeispiel für eine Filtervorrichtung gemäß der Erfindung näher erörtert, welches in der Zeichnung beschrieben ist.

Die Figur zeigt schematisch einen Aufbau einer in einem Abluftkanal 1 angeordneten Filtervorrichtung. Die Filtervorrichtung weist in Strömungsrichtung zunächst einen Partikelfilter 2 auf, bei welchem die Abluft eine Schicht aus einem mindestens teilweise offenporigen, hydrophilen Schaumstoff durchströmen muss. In dem Partikelfilter 2 werden Wasserdampf- und Fetttröpfchen zusammen mit anderen teilchenförmigen Verunreinigungen aus der Abluftströmung abgeschieden. Die meisten Geruchsstoffe können von solchen mechanischen Partikelfiltern jedoch nicht erfasst und herausgefiltert werden.

Anschließend wird die Abluft durch eine Energie zuführende Vorrichtung 3 geführt, die in Strömungsrichtung ausgerichtet eine erste Plattenelektrode 4 und im Abstand dazu eine zweite Plattenelektrode 5 aufweist, so dass die Luftströmung zwischen beiden Plattenelektroden 4, 5 hindurchströmen kann. Eine oder beide Plattenelektroden 4, 5 können auch als Teil des Abluftkanals 1 ausgestaltet sein und müssen nicht zwingend eine Veränderung des Querschnitts des Abluftkanals 1 bewirken. Mit einem Hochspannungstrafo 6 wird zwischen den beiden Plattenelektroden 4, 5 eine Wechselspannung angelegt. Die Frequenz der Wechselspannung beträgt 50 Hz, wobei auch andere Frequenzen, insbesondere höhere Frequenzen denkbar sind und für einzelne Anwendungen geeignet sein können.

Der durch die Energie zuführende Vorrichtung 3 hindurchgeführte Luftstrom wird darin zumindest teilweise ionisiert bzw. dissoziiert.

Anschließend wird die Luftströmung durch einen Geruchsfilter 7 geführt. Der Geruchsfilter 7 beinhaltet als Filtermaterial 8 Aktivkohle. In dem Geruchsfilter 7 werden nicht nur bislang noch nicht aus der Luftströmung herausgefilterte Teilchen, insbesondere Geruchsstoffe, sondern auch durch die Energie zuführende Vorrichtung 3 erzeugte Reaktionsprodukte, wie beispielsweise Ozon, aufgefangen und herausgefiltert. Durch die kurz vorher erzeugten, in dem Geruchsfilter 7 gesammelten hoch reaktiven Reaktionsprodukte werden weitere Reaktionen in dem Geruchsfilter 7 begünstigt, die zu einer zusätzlichen Verminderung und Herausfilterung von Geruchsstoffen führen.

Die Aktivkohle kann als Plattenmodul, als Schüttfilter oder als Patrone in dem Geruchsfilter 7 ausgestaltet sein. Anstelle der in der Figur dargestellten Anordnung von zwei Plattenelektroden 4, 5 können auch mehrere Elektroden übereinander angeordnet sein. Auch kann die Energie zuführende Vorrichtung 3 auch eine Vorrichtung zur Erzeugung eines Plasmas sein oder eine UV-Strahlungsquelle aufweisen.

## Patentansprüche

1. Verfahren zur Filterung von Gerüchen aus einer Luftströmung zur Anwendung mit einer Dunstabzugshaube, wobei ein Geruchsstoffe enthaltender Luftstrom durch eine Filtervorrichtung mit einem Geruchsfilter (7) hindurchgeführt wird und der Geruchsfilter (7) Aktivkohle als Filtermaterial aufweist, wobei in der Luftströmung vor dem Eintritt in den Geruchsfilter (7) ein Plasma erzeugt wird und dass zumindest ein Teil der in der Luftströmung enthaltenen Teilchen vor dem Eintritt in den Geruchsfilter (7) dissoziiert wird, **dadurch gekennzeichnet, dass** die Luftströmung zuerst durch einen mechanischen Partikelfilter (2) hindurchgeführt wird, bevor eine zumindest teilweise Dissoziation der darin enthaltenen Teilchen bewirkt wird und anschließend die Luftströmung in den Geruchsfilter (7) eintritt.

2. Verfahren nach Anspruch 1, **dadurch gekennzeichnet, dass** in der Luftströmung vor dem Eintritt in den Geruchsfilter (7) dielektrisch behinderte elektrische Entladungen erzeugt werden.

3. Filtervorrichtung zur Verwendung in einer Dunstabzugshaube für eine Luftströmung mit einem Geruchsfilter (7), wobei der Geruchsfilter (7) Aktivkohle als Filtermaterial aufweist, wobei die Filtervorrichtung eine Energie zuführende Vorrichtung (3) zur Erzeugung eines Plasmas zum Zwecke der Dissoziation der in der Luftströmung enthaltenen Teilchen aufweist, die entlang der Luftströmung vor dem Geruchsfilter (7) angeordnet ist, **dadurch gekennzeichnet, dass** die Filtervorrichtung einen mechanischen Partikelfilter (2) aufweist, der in Strömungsrichtung vor der Energie zuführenden Vorrichtung (3) zur Erzeugung eines Plasmas und dem nachfolgenden Geruchsfilter (7) angeordnet ist.

4. Filtervorrichtung nach Anspruch 3, **dadurch gekennzeichnet, dass** die Energie zuführende Vorrichtung (3) zwei im Abstand zueinander angeordnete Plattenelektroden (4, 5) aufweist, zwischen denen eine Wechselspannung anlegbar ist.

5. Filtervorrichtung nach Anspruch 3 oder 4, **dadurch gekennzeichnet, dass** die Energie zuführende Vorrichtung (3) zwei im Abstand zueinander angeordnete Plattenelektroden (4, 5) aufweist, zwischen denen eine pulsierende Gleichspannung oder eine Gleichspannung mit einer überlagerten Wechselspannung anlegbar ist.

6. Filtervorrichtung nach Anspruch 4 oder 5, **dadurch gekennzeichnet, dass** zwischen den Elektroden (4, 5) ein die Entladung behinderndes dielektrisches Material angeordnet ist.

7. Filtervorrichtung nach einem der Ansprüche 3 bis 6, **dadurch gekennzeichnet, dass** der Partikelfilter (2) ein mindestens teilweise offenporiger, hydrophiler Schaumstoff ist.

8. Filtervorrichtung nach einem der Ansprüche 3 bis 7, **dadurch gekennzeichnet, dass** der Partikelfilter (2) ein Staubfilter ist.

## Claims

1. Method for filtering odours out of an air flow, for use with a fume extractor hood, wherein an air flow containing odorous substances is conducted through a filter device with an odour filter (7), and the odour filter (7) has activated carbon as a filter material, wherein a plasma is generated in the air flow upstream of the inlet into the odour filter (7), and in that at least some of the particles contained in the air flow are dissociated before entering the odour filter (7), **characterized in that** the air flow is firstly conducted through a mechanical particle filter (2) before an at least partial dissociation of the particles contained in said air flow is effected and subsequently the air flow enters into the odour filter (7).

2. Method according to Claim 1, **characterized in that** dielectric barrier electric discharges are generated in the air flow upstream of the inlet into the odour filter (7).

3. Filter device for use in a fume extraction hood for an air flow, having an odour filter (7), wherein the odour filter (7) has activated carbon as filter material, wherein the filter device has an energy-supplying device (3) for generating a plasma for dissociation of the particles contained in the air flow, said device being arranged along the air flow upstream of the odour filter (7), **characterized in that** the filter device has a mechanical particle filter (2) which, as viewed in the flow direction, is arranged upstream of the energy-supplying device (3) for generating a plasma and of the downstream odour filter (7).

4. Filter device according to Claim 3, **characterized in that** the energy-supplying device (3) has two plate electrodes (4, 5) which are arranged at a distance from one another and between which can be applied an alternating-current voltage.

5. Filter device according to Claim 3 or 4, **characterized in that** the energy-supplying device (3) has two plate electrodes (4, 5) which are arranged at a distance from one another and between which can be applied a pulsed direct-current voltage or a direct-current voltage with a superposed alternating-current voltage.

6. Filter device according to Claim 4 or 5, **characterized in that** a dielectric material which forms a barrier to the discharge is arranged between the electrodes (4, 5).

7. Filter device according to one of Claims 3 to 6, **characterized in that** the particle filter (2) is an at least partially open-pored, hydrophilic foamed material.

8. Filter device according to one of Claims 3 to 7, **characterized in that** the particle filter (2) is a dust filter.

## Revendications

1. Procédé pour extraire par filtration des odeurs d'un flux d'air destiné à être employé avec une hotte d'aspiration, dans lequel un flux d'air comportant des subtances odorantes est passé à travers un dispositif de filtration muni d'un filtre anti-odeurs (7) et le filtre anti-odeurs (7) présente du charbon actif comme matériau filtrant, sachant que dans le flux d'air, un plasma est produit avant l'entrée dans le filtre anti-odeurs (7) et qu'au moins une partie des particules contenues dans le flux d'air sont dissociées avant l'entrée dans le filtre anti-odeurs (7), **caractérisé en ce que** le flux d'air traverse d'abord un filtre à particules (2) mécanique avant qu'une dissociation au moins partielle des particules contenues dedans ne soit effectuée et qu'enfin le flux d'air pénètre dans le filtre anti-odeurs (7).

2. Procédé selon la revendication 1, **caractérisé en ce que** des décharges électriques à inhibition diélectrique sont produites dans le flux d'air avant l'entrée dans le filtre anti-odeurs (7).

3. Dispositif de filtration destiné à être employé dans une hotte d'aspiration pour un flux d'air comprenant un filtre anti-odeurs (7), dans lequel le filtre anti-odeurs (7) présente du charbon actif comme matériau filtrant, dans lequel le dispositif de filtration présente un dispositif (3) amenant de l'énergie pour produire un plasma pour la dissociation des particules contenues dans le flux d'air qui sont disposées le long du flux d'air avant le filtre anti-odeurs (7), **caractérisé en ce que** le dispositif de filtration présente un filtre à particules (2) mécanique qui est disposé dans le flux d'air avant le dispositif (3) amenant de l'énergie pour produire un plasma et le filtre à odeurs (7) en aval.

4. Dispositif de filtration selon la revendication 3, **caractérisé en ce que** le dispositif (3) amenant de l'énergie présente deux électrodes à plaque (4, 5) disposées à distance l'une de l'autre, entre lesquelles une tension alternative peut être mise.

5. Dispositif de filtration selon la revendication 3 ou 4, **caractérisé en ce que** le dispositif (3) amenant de l'énergie présente deux électrodes à plaque (4, 5) disposées à distance l'une de l'autre, entre lesquelles une tension continue à pulsations ou une tension continue avec une tension alternative ondulée peut être mise.

6. Dispositif de filtration selon la revendication 4 ou 5, **caractérisé en ce qu'**un matériau diélectrique inhibant la décharge est disposé entre les électrodes (4, 5).

7. Dispositif de filtration selon l'une des revendications 3 à 6, **caractérisé en ce que** le filtre à particules (2) est une mousse hydrophile au moins à pores partiellement ouvertes.

8. Dispositif de filtration selon l'une des revendications 3 à 7, **caractérisé en ce que** le filtre à particules (2) est un filtre à poussières.
